(19) Europäisches Patentamt
European Patent Office
Office européen des brevets

(11) **EP 4 502 149 A1**

(12) **EUROPEAN PATENT APPLICATION**
published in accordance with Art. 153(4) EPC

(43) Date of publication:
**05.02.2025 Bulletin 2025/06**

(21) Application number: **23780837.3**

(22) Date of filing: **30.03.2023**

(51) International Patent Classification (IPC):
***C12N 5/10*** (2006.01)    ***C12P 21/08*** (2006.01)

(52) Cooperative Patent Classification (CPC):
**C07K 16/00; C12N 5/10**

(86) International application number:
**PCT/JP2023/013100**

(87) International publication number:
**WO 2023/190829 (05.10.2023 Gazette 2023/40)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **31.03.2022 JP 2022058476**

(71) Applicant: **FUJIFILM Corporation
Tokyo 106-8620 (JP)**

(72) Inventors:
• **KUROSAWA, Takashi
Ashigarakami-gun, Kanagawa 258-8577 (JP)**
• **INADA, Atsushi
Ashigarakami-gun, Kanagawa 258-8577 (JP)**
• **NAKAI, Shinichi
Ashigarakami-gun, Kanagawa 258-8577 (JP)**

(74) Representative: **Hoffmann Eitle
Patent- und Rechtsanwälte PartmbB
Arabellastraße 30
81925 München (DE)**

(54) **PRODUCT MANUFACTURING METHOD AND PRODUCT**

(57)    An object of the present invention is to provide a method that makes it possible to suppress membrane clogging in a production method for a product by cell culture, and a product that is produced by the method. According to the present invention, there is provided a production method for a product, which includes culturing cells, where the production method for a product is perfusion culture in which a cell density at a time of production of the product is $80 \times 10^6$ cells/mL or more and $300 \times 10^6$ cells/mL or less, where a period of the perfusion culture is 13 days or more and 500 days or less, and in a period of at least one day or more in the period of the perfusion culture, X [mol/L/day], which is an alkali addition rate per day, is controlled in a range of $0 \le X < 0.029$, where a pH of an aqueous alkali solution to be added satisfies $7 < pH < 13$.

EP 4 502 149 A1

## Description

Technical Field

The present invention relates to a production method for a product, which includes culturing cells, where the production method for a product is a method in which an alkali addition rate and a pH of an aqueous alkali solution are controlled. The present invention further relates to a product produced by the above-described production method.

Background Art

Cell culture is carried out for the intended purpose, for example, increasing the number of cells having useful properties or causing cells to produce a product. For example, Patent Document 1 discloses a method of adjusting a high-mannose glycoform content of a recombinant protein containing an Fc region of immunoglobulin during a mammalian cell culture process, where the method includes establishing a mammalian cell culture that allows an expression of a recombinant protein in a serum-free culture medium in a bioreactor, maintaining mammalian cells during a production phase, and bringing the cell into contact with monensin.

In addition, Patent Document 2 discloses a method that includes, in a fed-batch culture method for culturing mammalian cells which includes controlling a pH using a pH set point, a first culture step which includes seeding mammalian cells at a first pH in a culture medium and culturing the cells at the first pH, where the first culture step is such that the pH set point is maintained at the first pH; and a second culture step which includes culturing the cells at a second pH higher than the first pH, where the second culture step is such that the set point is maintained at the second pH, where the method is such that the second pH is higher than the first pH by at least 0.1 pH units, and the second culture step has a duration of at least 6 hours.

Further, Patent Document 3 discloses a method for culturing an immortalized human blood cell, preferably, cells originating from myeloid leukemia, or a suspension of cells derived therefrom, where the method makes it possible to provide high productivity, high cell survival rate, and high proliferation rate, as well as high inter-batch consistency, and achieve scale-up without changing these parameters.

Prior Art Documents

Patent Documents

Patent Document 1: JP2016-534732A
Patent Document 2: JP2021-503916A
Patent Document 3: JP2014-500032A

## SUMMARY OF THE INVENTION

Object to be solved by the invention

An alternating tangential flow filtration (ATF) method that is generally used in perfusion culture is less likely to cause membrane clogging as compared with a tangential flow filtration (TFF) method due to a backwashing effect which is obtained by a liquid flowing back from a secondary side of a membrane to a primary side thereof. However, as developments in productivity improvement progress, densification has progressed regarding the cell density, and membrane clogging is an issue even in the ATF method. A general measure for suppressing membrane clogging is to increase the membrane area. However, there is a problem in that commercially available membranes have a small area and devices are enlarged in size.

An object to be achieved by the present invention is to provide a method that makes it possible to suppress membrane clogging in a production method for a product by cell culture. Further, another object to be achieved by the present invention is to provide a product that is produced by the method.

Means for solving the object

As a result of diligent studies to achieve the above objects, the inventors of the present invention found that membrane clogging can be suppressed by controlling a pH and an addition rate of an aqueous alkali solution that is used for pH control, thereby completing the present invention.

That is, according to the present invention, the following inventions are provided.

<1> A production method for a product, comprising:

culturing cells,
in which the production method for a product is perfusion culture in which a cell density at a time of production of the product is $80 \times 10^6$ cells/mL or more and $300 \times 10^6$ cells/mL or less, where a period of the perfusion culture is 13 days or more and 500 days or less, and
in a period of at least one day or more in the period of the perfusion culture, X [mol/L/day], which is an alkali addition rate per day, is controlled in a range of $0 \leq X < 0.029$, where a pH of an aqueous alkali solution to be added satisfies $7 < pH < 13$.

<2> The production method for a product according to <1>, in which the cell density in the perfusion culture is $100 \times 10^6$ cells/mL or more and $300 \times 10^6$ cells/mL or less.

<3> The production method for a product according to <1> or <2>, in which the period of the perfusion culture is 18 days or more and 500 days or less.

<4> The production method for a product according to any one of <1> to <3>, in which a period of culture for the production of the product is 5 days or more and 450 days or less.

<5> The production method for a product according to any one of <1> to <4>, in which in a period of at least 13 days or more in the period of the perfusion culture, X [mol/L/day], which is the alkali addition rate per day, is controlled in a range of $0 \leq X < 0.008$.

<6> The production method for a product according to any one of <1> to <5>, in which in a period of at least 13 days or more in the period of the perfusion culture, X [mol/L/day], which is the alkali addition rate per day, is 0.

<7> The production method for a product according to any one of <1> to <6>, in which in a period of 50% or more in the period of the perfusion culture, X [mol/L/day], which is the alkali addition rate per day, is controlled in a range of $0 \leq X < 0.029$.

<8> The production method for a product according to any one of <1> to <7>, in which a continuous separation method for a cell culture solution in the perfusion culture is membrane filtration.

<9> The production method for a product according to <8>, in which the membrane filtration is alternating tangential flow filtration, which is referred to as ATF.

<10> The production method for a product according to <8> or <9>, in which Y [L/m²/hour], which is a flux during filtration, satisfies $0 < Y \leq 10$.

<11> The production method for a product according to any one of <1> to <10>, in which an average pH of a culture solution during the culturing is 6.7 to 7.2.

<12> The production method for a product according to any one of <1> to <11>, in which a minimum pH of a culture solution during the culturing is 6.6 or more.

<13> The production method for a product according to any one of <1> to <12>, in which a pH of a culture solution during the culturing is controlled by automatically adding an aqueous alkali solution while subjecting a pH in the culture solution to an in-line measurement.

<14> The production method for a product according to any one of <1> to <13>, in which in the perfusion culture, after a cell density reaches a target cell density, which is $80 \times 10^6$ cells/mL or more, a culture solution containing cells is extracted to maintain the cell density within $\pm 40\%$ of the target cell density, and the product is recovered in a period in which the cell density is maintained within $\pm 40\%$ of the target cell density.

<15> The production method for a product according to any one of <1> to <13>, further comprising:
extracting a culture solution containing cells at least once or more a day in the perfusion culture after a cell density reaches a target cell density, which is $80 \times 10^6$ cells/mL or more, and then adjusting the cell density to be within $\pm 10\%$ of the target cell density.

<16> The production method for a product according to any one of <1> to <15>, in which a dissolved $CO_2$ concentration in a period in which the cell density is maintained at $80 \times 10^6$ cells/mL or more is 60 to 180 mmHg.

<17> The production method for a product according to any one of <1> to <16>, in which the alkali is $NaHCO_3$ and/or $Na_2CO_3$.

<18> The production method for a product according to any one of <1> to <17>, in which the alkali is $NaHCO_3$.

<19> The production method for a product according to any one of <1> to <18>, in which a concentration Z [mol/L] of the aqueous alkali solution satisfies $0 < Z < 5$.

<20> The production method for a product according to any one of <1> to <19>, in which a pH of a culture medium to be added in the perfusion culture is 7.0 to 8.0.

<21> The production method for a product according to any one of <1> to <20>, in which in a case where a pH of a culture solution decreases in a case where the culturing is carried out without adding an alkali, a culture medium to which an alkali is added is supplied to control the pH of the culture solution.

<22> The production method for a product according to any one of <1> to <21>, further comprising:

supplying a culture medium to which an alkali of $1.0 \times 10^{-3}$ to 2.0 mol/L is added, in a case where a pH of a culture solution is less than 6.85.

<23> The production method for a product according to any one of <1> to <22>, in which an anti-foaming agent containing dimethicone is added in the perfusion culture, and X [mol/L/day], which is an addition rate of the alkali, and B [g/L/day], which is an addition rate of the dimethicone, satisfy B < -0.79X + 0.0228.

<24> The production method for a product according to any one of <1> to <23>, in which alkali addition is not carried out in an entire period of the perfusion culture.

<25> The production method for a product according to any one of <1> to <24> in which the cell is an animal cell.

<26> The production method for a product according to any one of <1> to <25>, in which the cell is a CHO cell.

<27> The production method for a product according to any one of <1> to <26>, in which the product is an antibody.

<28> A product that is produced by the production method for a product according to any one of <1> to <27>.

Effect of the invention

According to the present invention, it is possible to suppress membrane clogging in perfusion culture.

**BRIEF DESCRIPTION OF THE DRAWINGS**

[Fig. 1] Fig. 1 shows a cell culture device. The entire device shown in Fig. 1 is a cell culture device.

[Fig. 2] Fig. 2 shows a relationship between an alkali addition rate and the number of days taken from the start of culture to the membrane clogging.

[Fig. 3] Fig. 3 shows a relationship between an alkali addition rate and a fine particle density.

[Fig. 4] Fig. 4 shows a relationship between an alkali addition rate and LDH.

[Fig. 5] Fig. 5 shows a relationship between an alkali addition rate, a dimethicone addition rate, and the number of days taken from the start of culture to the membrane clogging.

**EMBODIMENTS FOR CARRYING OUT THE INVENTION**

Hereinafter, the contents of the present invention will be described in detail. In the present specification, a numerical value range indicated using "to" means a range including numerical values described before and after "to" as a minimum value and a maximum value, respectively.

The present invention relates to a production method for a product, which includes culturing cells, where the production method for a product is perfusion culture in which a cell density at a time of production of the product is $80 \times 10^6$ cells/mL or more and $300 \times 10^6$ cells/mL or less, where a period of the perfusion culture is 13 days or more and 500 days or less, and in a period of at least one day or more in the period of the perfusion culture, X [mol/L/day], which is an alkali addition rate per day, is controlled in a range of $0 \le X < 0.029$, where a pH of an aqueous alkali solution to be added satisfies 7 < pH < 13.

According to the present invention, it is possible to suppress membrane clogging in perfusion culture. According to the present invention, it is possible to improve productivity in perfusion culture using a membrane, particularly such as ATF/TFF.

Here, the alkali addition per day means the alkali addition that is carried out by directly adding an aqueous alkali solution to a culture solution in addition to a culture medium, and even in a case where a culture medium to be added to a culture tank during perfusion culture contains an alkali, this case does not correspond to the case of the alkali addition described herein. In addition, in the present specification, the alkali addition is intended to be a direct addition to a culture solution in a case where there is no description regarding addition to a culture medium or the like.

In the present invention, the cell density at the time of the production of the product is $80 \times 10^6$ cells/mL or more, and it is preferably $80 \times 10^6$ to $300 \times 10^6$ cells/mL, more preferably $90 \times 10^6$ to $300 \times 10^6$ cells/mL, still more preferably $100 \times 10^6$ to $300 \times 10^6$ cells/mL, even still more preferably $110 \times 10^6$ to $300 \times 10^6$ cells/mL, and even further still more preferably $120 \times 10^6$ to $300 \times 10^6$ cells/mL. M may be used to denote $10^6$. In particular, in a case where the cell density is set to $120 \times 10^6$ to $300 \times 10^6$ cells/mL, membrane clogging is remarkably observed, and thus the effect of the present invention is remarkably obtained.

Preferably, in the present invention, in the perfusion culture described later, it is possible to extract a culture solution containing cells after a cell density reaches a target cell density, which is $80 \times 10^6$ cells/mL or more, thereby maintaining the cell density within $\pm 40\%$ of the target cell density, and to recover the product in a period in which the cell density is maintained within ± 40% of the target cell density.

Preferably, it is possible to extract a culture solution containing cells at least once or more a day in the perfusion culture after a cell density reaches a target cell density, which is $80 \times 10^6$ cells/mL or more, and then adjust the cell density to be within ± 10% of the target cell density.

In the present invention, the mode of the method of culturing cells is perfusion culture. The perfusion culture is a culture method in which a fresh culture medium is supplied into a cell culture solution and a part of the culture medium in which cells are cultured is removed. In a case where this perfusion culture is carried out, it is possible to remove, from a culture tank, metabolic decomposition products discharged from cells. In the perfusion culture, it is possible to recover a liquid obtained by continuously separating cells in a culture solution while continuously supplying a culture medium to a culture tank.

In general, the perfusion culture makes it possible to achieve a high viable cell density. A typical perfusion culture begins with a batch culture that continues for 1 or 2 days, thereafter a fresh supply culture medium is added to the culture continuously, stepwise, and/or intermittently, and the used culture medium is removed at the same time. In the perfusion culture, it is also possible to separate cells by using methods such as sedimentation, centrifugation, and filtration and remove the used culture medium while maintaining the viable cell density. The advantage of the perfusion culture is that the culture in which a target protein is produced is maintained for a long period as compared with the batch culture method or the fed-batch culture.

Perfusion may be any form of being continuous, stepwise, intermittent, or a combination thereof. A continuous form is preferable. Animal cells are retained in the culture and the used culture medium that is removed may substantially not include cells or may have much fewer cells than the culture. A product that is expressed by cell culture can be retained in the culture or recovered by the selection of the membrane pore diameter.

The continuous separation method for a cell culture solution in the perfusion culture is preferably membrane filtration and more preferably alternating tangential flow filtration (ATF).

Y [L/m²/hour], which is a flux during filtration, preferably satisfies $0 < Y \le 10$, more preferably satisfies $0 < Y \le 5$, and still more preferably satisfies $0 < Y \le 2$.

The flux during filtration is an amount of a culture solution that permeates through a membrane per unit time and per unit filtration area, and it is defined by the following expression.

$$\text{Flux during filtration [L/m}^2\text{/hour]} = \frac{\text{Flow rate when passing through filtration membrane [L/hour]}}{\text{Area of filtration membrane [m}^2\text{]}}$$

The perfusion ratio is not particularly limited; however, it is generally 0.3 vvd to 5.0 vvd, preferably 0.5 vvd to 2.0 vvd, and more preferably 0.5 vvd to 1.4 vvd. vvd means an amount required for exchanging a cell culture solution per the volume of the cell culture solution for one day with a fresh culture medium, that is, it is "volume of supply culture medium/volume of culture solution/Day".

A method for extracting a product from a culture solution can be carried out by draining the product from the secondary side of the filtration membrane with a pump; however, another available liquid feeding means may be used. The extracted culture solution is subjected to treatments such as product recovery and removal of the dead cells. In addition, the extracted culture solution may be partially discarded or returned to the culture container after treatments such as product recovery and removal of the dead cells. In a case where a loss of the culture solution occurs due to the above treatments, the loss can be compensated, for example, by supplying a fresh culture medium to the culture container.

In the perfusion culture, it is possible to extract a culture solution containing cells at least once or more a day after a cell density reaches a target cell density, which is $80 \times 10^6$ cells/mL or more, and then adjust the cell density to be within ± 10% of the target cell density.

Extracting a part of a culture solution together with cells so that the viable cell density during the culturing does not become excessive, thereby reducing the viable cell density is referred to as cell bleeding, and the amount of the culture solution can be maintained by adding the same amount of a fresh culture medium as the amount of the extracted culture solution. The term "once or more a day" includes a case of continuously and automatically carrying out cell bleeding.

The period of the perfusion culture (this period is a culture period from the start of the perfusion and is a period including a period before cells reach a high density) is not particularly limited; however, it is generally 1 day or more and 1,000 days or less, preferably 7 days or more and 1,000 days or less, more preferably 18 days or more and 500 days or less, still more preferably 25 days or more and 200 days or less, and even still more preferably 30 days or more and 100 days or less. The perfusion may be started 2 days after seeding with the day of seeding as the 0th day.

The period of culture for the production of the product in which the cell density is $80 \times 10^6$ cells/mL or more is preferably 5 days or more and 990 days or less and may be 5 days or more and 450 days or less, and it is more preferably 10 days or more and 450 days or less, still more preferably 15 days or more and 190 days or less, and even still more preferably 20 days or more and 90 days or less.

In the present invention, in a period of at least 13 days or more in the period of the perfusion culture, X [mol/L/day], which is an alkali addition rate per day, is controlled in a range of $0 \leq X < 0.029$, preferably controlled in a range of $0 \leq X < 0.015$, more preferably controlled in a range of $0 \leq X < 0.0127$, and still more preferably controlled in a range of $0 \leq X < 0.008$.

In the present invention, X [mol/L/day], which is an alkali addition rate per day, may be 0 in a period of at least 13 days or more in the period of the perfusion culture. The case where X is 0 means that the alkali addition is not carried out from a line other than the culture medium supply line.

In the present invention, X [mol/L/day], which is an alkali addition rate per day, is preferably controlled in a range of $0 \leq X < 0.029$ in a period of 50% or more in the period of the perfusion culture (preferably 70% or more, more preferably 80% or more, still more preferably 90% or more, and most preferably 100%).

The alkali is not particularly limited; however, it is preferably $Na_2CO_3$, $NaOH$, or $NaHCO_3$, more preferably $NaHCO_3$ and/or $Na_2CO_3$, and particularly preferably $NaHCO_3$. The alkali can be added as an aqueous solution (for example, an $Na_2CO_3$ aqueous solution, an $NaOH$ aqueous solution, or an $NaHCO_3$ aqueous solution).

In a case where $Na_2CO_3$ or $NaHCO_3$ is added to a cell culture solution, a cell culture solution to which $NaHCO_3$ is added tends to have a lower viscosity. That is, it can be said that $NaHCO_3$ has a weak action of gelating the culture solution as compared with $Na_2CO_3$ and thus is likely to suppress membrane clogging, and thus it is particularly preferable to use $NaHCO_3$.

The time of the start of alkali addition is 7 days or later after the start of culture, preferably 6 days or later after the start of culture, and more preferably 5 days or later after the start of culture.

The pH of the aqueous alkali solution to be added satisfies $7 < pH < 13$, preferably satisfies $7.5 < pH < 12$, more preferably satisfies $7.5 < pH < 10$, and still more preferably satisfies $8 < pH < 10$.

The pH of the aqueous alkali solution is measured at a time when the alkali has dissolved in water. The pH can be measured using, in general, a commercially available pH sensor. Examples thereof include a pH meter (Seven Excellence, Seven Direct, Five Easy) manufactured by METTLER TOLEDO.

The concentration Z [mol/L] of the aqueous alkali solution to be added preferably satisfies $0 < Z < 5$, more preferably satisfies $0 < Z < 3$, and still more preferably satisfies $0 < Z < 2$.

In the entire period of the perfusion culture, it is possible to carry out culture without adding alkalis at all. However, in a case where alkali is not added, the pH of the culture solution may be decreased, which deteriorates the quality of the product (Table 3). In such a case, a method of maintaining the quality of the product while not accelerating membrane clogging is required.

It is possible to improve the antibody quality while suppressing the membrane clogging by adding an alkali to the culture medium at N [mol/L] in a case where the pH of the culture solution is less than a certain value M. That is, in a case where a pH of a culture solution decreases in a case where the culturing is carried out without adding an alkali, it is possible to control the pH of the culture solution by supplying a culture medium to which an alkali has been added. The denominator of N is the volume of the culture medium after preparation. In this case, a culture medium in which the amount of alkali is increased is newly prepared, and a change is made so that a container containing the old culture medium (culture medium being supplied to the culture tank) and a tube connected to the culture tank are aseptically connected to a container containing a new culture medium by using a thermal welding machine, whereby the supply is started. Alternatively, an alkali may be aseptically added to a container containing a culture medium, where the container is connected the culture tank. The value M of the pH of the culture solution is 6.85, preferably 6.80, and more preferably 6.75.

The N [mol/L], which is an amount of an alkali to be added to the culture medium, is $1.0 \times 10^{-3}$ to 2.0 mol/L, preferably $2.0 \times 10^{-3}$ to 1.0 mol/L, and more preferably $1.0 \times 10^{-2}$ to $2.0 \times 10^{-1}$ mol/L. Here, the alkali to be added to the culture medium does not mean an alkali that is directly added to the culture solution but means an alkali that is added to the culture medium before being supplied to the culture tank. For example, in a case where a pH of a culture solution is less than 6.85, it is possible to supply a culture medium to which an alkali of $1.0 \times 10^{-3}$ to 2.0 mol/L has been added

In a case where a pH of a culture solution decreases in a case where the culturing is carried out without directly adding an alkali to the culture solution as described above, it is possible to control the pH of the culture solution by supplying a culture medium having an increased pH. The culture medium having an increased pH may be prepared in advance, or the preparation thereof may be started in a case where a change in the pH of the culture solution is observed. The perfusion culture can be carried out by switching the supply culture medium to a culture medium having an increased pH, within 24 hours after the pH starts to fall below M. The time is preferably within 12 hours or less, more preferably within 6 hours or less, still more preferably within 3 hours or less, even still more preferably within 1 hour or less, even still more preferably within 30 minutes or less, and further even still more preferably within 5 minutes or less.

The liquid amount to be prepared is preferably 0.5 to 10 times the amount of the culture solution. The alkali to be added to the culture medium is not particularly limited; however, it is preferably $Na_2CO_3$, $NaOH$, or $NaHCO_3$, more preferably $NaHCO_3$ and/or $Na_2CO_3$, and particularly preferably $NaHCO_3$.

The pH of the culture medium to be added in the perfusion culture is preferably 7.0 to 8.0, more preferably 7.0 to 7.8, and still more preferably 7.0 to 7.6.

The pH of the culture medium can be measured using, in general, a commercially available pH sensor after storing the culture medium for 1 day in incubation at 5% $CO_2$ and 37°C. Examples thereof include a pH meter (Seven Excellence, Seven Direct, Five Easy) manufactured by METTLER TOLEDO.

The average pH of the culture solution during the culturing is preferably 6.7 to 7.2 and more preferably 6.8 to 7.0.

The minimum pH of the culture solution during the culturing is preferably 6.6 or more, more preferably 6.7 or more, and still more preferably 6.8 or more.

Preferably, it is possible to control a pH of a culture solution during the culturing by automatically adding an aqueous alkali solution while subjecting a pH in the culture solution to an in-line measurement.

Preferably, an anti-foaming agent can be added in the perfusion culture. The anti-foaming component of the anti-foaming agent is preferably silicone-based, and it is particularly preferably dimethicone. The anti-foaming component of the anti-foaming agent is preferably an anti-foaming component containing polydimethylsiloxane, and it is more preferably an anti-foaming component in which fine powder silica is contained in polydimethylsiloxane. As the anti-foaming agent, it is possible to use, for example, HyClone ADCF Antifoam Agent manufactured by Cytiva. More preferably, X [mol/L/day], which is an addition rate of the alkali, and B [g/L/day], which is an addition rate of the dimethicone per day, preferably satisfy B < -0.79X + 0.0228. In this case, B does not become a negative value. Therefore, X ≤ 0.0288.

The denominator of B is the volume of the culture tank and the culture solution contained in the ATF.

In a case where this relationship is satisfied, it is possible to more effectively suppress membrane clogging.

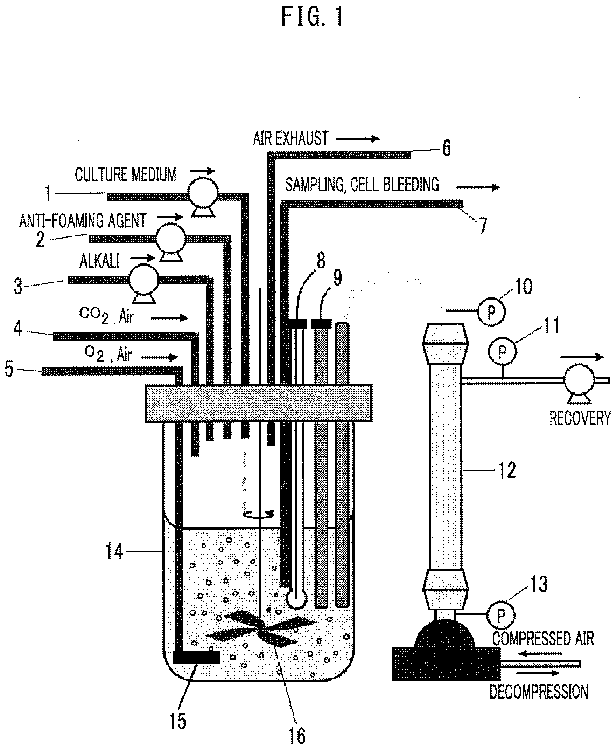

Fig. 1 shows one example of a cell culture device that can be used in the cell culture in the present invention. In Fig. 1, a culture container 14 is a container that accommodates a culture solution containing cells. Cells are cultured in the culture solution in the inside of the culture container 14.

The culture medium is supplied to the culture container from a culture medium supply pipe 1.

An anti-foaming agent for suppressing foaming is supplied from an anti-foaming agent supply pipe 2 to the culture container.

An alkali is supplied from an alkali supply pipe 3 to the culture container. In a case where the alkali is not added, the alkali supply pipe 3 may not be provided.

Carbon dioxide ($CO_2$) and air are introduced from an air supply pipe 4 into an upper part of the culture solution in the side of the culture container.

Oxygen ($O_2$) and/or air is sent from a sparger air supply pipe 5, and the oxygen and/or air is introduced into the culture solution through a sparger 15 having a pore diameter of 20 μm. The dissolved oxygen concentration inside the culture solution can be adjusted by the sparger 15. The sparger is not particularly limited; however, it is possible to use, for example, a sparger in which an average pore diameter of a gas release unit is 1 μm or more and 300 μm or less (for example, a pore diameter of 20 μm) and which releases a gas containing 30% by volume or more of oxygen.

An exhaust pipe 6 is a pipe for exhausting air, and an exhaust filter (not shown in the drawing) may be connected to a terminal thereof.

The sampling tube 7 is a pipe for collecting (sampling) the culture solution or extracting (cell bleeding) the culture solution. In a case where the cell is automatically and continuously subjected to cell bleeding, a pipe may be separately installed (not shown in the drawing).

A pH sensor 8 is mounted to come into contact with the culture solution.

A dissolved oxygen sensor 9 is mounted to come into contact with the culture solution.

Pressure sensors 10, 11, and 13 may be provided in the cell culture device.

A hollow fiber membrane 12 is installed in the cell culture device.

A stirring member having a stirring blade 16 may be provided inside the culture container 14. In a case where the stirring blade 16 is rotated, the culture solution inside the culture container 14 is stirred, and the homogeneity of the culture solution is maintained. In a case where the culture solution is stirred by the stirring blade 16, the bubbles released by the sparger are also stirred. The position of the stirring member having a stirring blade, the size of the stirring blade, and the like are not particularly limited and may be designed depending on the cell kind to be used, the amount of the culture solution, the amount of oxygen to be supplied, or the position, number, size, or the like of the sparger. Further, in order to quickly stir bubbles coming out of the sparger and suppress coalescence of the bubbles, it is preferable to dispose the stirring blade 16 at a position close to the sparger.

In the present invention, a cell suspension extracted from the culture container may be allowed to pass through a separation membrane to separate the cell suspension into a cell-containing liquid and a permeated solution. This operation can be carried out using a cell culture device. In this operation, the cell suspension extracted from the culture container is separated into a cell-containing liquid having a cell concentration higher than that of the cell suspension and a permeated solution having a cell concentration lower than that of the cell suspension. The cell concentration can be measured by a cell viability analyzer, Vi-CELL XR, manufactured by Beckman Coulter Life Sciences.

The membrane separation treatment step described above is preferably tangential filtration, more preferably alternating tangential flow (ATF) filtration or tangential flow filtration, and most preferably alternating tangential flow filtration. Examples of the filter that makes it possible to carry out alternating tangential flow filtration include SuATF10-S02PES and F2 RF02PES, which are manufactured by Repligen Corporation.

As the culture medium that is used for cell culture, a culture medium that is generally used for culturing animal cells can be used. For example, CD OptiCHO (manufactured by Thermo Fisher Scientific, Inc.), Dulbecco's modified Eagle medium (DMEM), Eagle minimum essential medium (MEM), RPMI-1640 medium, RPMI-1641 medium, F-12K medium, Ham's F12 medium, Iscove's modified Dulbecco's medium (IMDM), McCoy's 5A medium, Leibovitz's L-15 medium, and EX-CELL (trade mark) 300 series (JRH Biosciences), CHO-S-SFMII (Invitrogen), CHO-SF (Sigma-Aldrich Co. LLC), CD-CHO (Invitrogen), ISCHO-V (FUJIFILM Irvine Scientific), PF-ACF-CHO (Sigma-Aldrich Co. LLC), and the like can be used. Alternatively, a homemade culture medium may be used.

Serum such as fetal calf serum (FCS) may be added to the culture medium, or serum may not be added thereto. The culture medium may be supplemented with additional components such as an amino acid, salts, a sugar, a vitamin, a hormone, a growth factor, a buffer solution, an antibiotic, a lipid, a trace element, and a hydrolysate of a plant protein. A protein-free culture medium can also be used.

The culture temperature is generally 30°C to 40°C, preferably 32°C to 39°C, and more preferably 36°C to 38°C, and the culture temperature may be changed during the culturing.

The culture can be carried out in an atmosphere having a $CO_2$ concentration of 0% to 40% by volume, preferably 2 to 25% by volume, and more preferably 3 to 20% by volume.

The amount of the culture solution is preferably 0.5 L or more, more preferably 50 L or more, and still more preferably 200 L or more.

In the culture, the culture medium can be replaced, aerated, and stirred as necessary. In a case of carrying out stirring of the culture solution, the rotation speed of stirring is not particularly limited; however, the stirring power per unit volume is generally 10 to 300 kW/m$^3$, preferably 20 to 200 kW/m$^3$, and more preferably 30 to 100 kW/m$^3$.

The dissolved oxygen concentration in the culture solution can be appropriately set and is not particularly limited; however, it is generally 10% to 100% and preferably 30% to 90%.

In addition, a dissolved $CO_2$ concentration in a period in which the cell density is maintained at $80 \times 10^6$ cells/mL or more is preferably 60 to 180 mmHg, more preferably 80 to 160 mmHg, and still more preferably 100 to 140 mmHg.

The cell culture can be carried out using a cell culture device having the configuration described above in the present specification. The cell culture device may be any one of a fermenter tank type culture device, an air lift type culture device, a culture flask type culture device, a spinner flask type culture device, a microcarrier type culture device, a fluidized bed type culture device, a hollow fiber type culture device, a roller bottle type culture device, a filling tank type culture device, or the like. The culture container is preferably a single-use culture tank from the viewpoint of homogenizing the culture environment or the like.

The viscosity of the culture solution for producing a product, in which the cell density is $80 \times 10^6$ cells/mL or more, is preferably 1.2 mPa·s or more and less than 15 mPa·s, more preferably 1.4 mPa·s or more and less than 12 mPa·s, still more preferably 1.6 mPa·s or more and less than 10 mPa·s, particularly preferably 1.6 mPa·s or more and less than 5 mPa·s, and most preferably 1.6 mPa·s or more and less than 3 mPa·s.

The kind of the cell in the present invention is not particularly limited; however, examples thereof include eukaryotic cells such as an animal cell, a plant cell, and yeast, prokaryotic cells such as Bacillus subtilis, and Escherichia coli. The cell is preferably an animal cell (more preferably a mammalian cell) or an insect cell, and it is most preferably a mammalian cell. The cell may be a primary cell or a cell established as a cell line.

Examples of the cell include a Chinese hamster ovary (CHO) cell, a HEK cell (a cell derived from the human embryonic kidney), a BHK cell, a 293 cell, a C127 cell, a myeloma cell (such as an NS0 cell), a PerC6 cell, an SP2/0 cell, a hybridoma cell, a COS cell (a cell derived from the kidney of the African green monkey), a 3T3 cell, a HeLa cell, a Vero cell (a renal epithelial cell of the African green monkey), a MDCK cell (a cell derived from a canine kidney renal tubular epithelial cell), a PC12 cell, and a WI38 cell. The cell may be a stem cell such as an embryonic stem cell (ES cell) or an induced pluripotent stem cell (iPS cell). Among these, a CHO cell, a HEK cell, a BHK cell, or a hybridoma is preferable, where a CHO cell or a HEK cell is more preferable, and a CHO cell is most preferable. The CHO cell is widely used for the production of recombinant proteins such as a cytokine, a coagulation factor, and an antibody. It is preferable to use a CHO cell deficient in dihydrofolate reductase (DHFR), and as a DHFR-deficient CHO cell, it is possible to use, for example, CHO-DG44.

It is preferable that the cell viability is high; however, it is preferably 85% or more, more preferably 90% or more, particularly preferably 95% or more, and most preferably 99% or more.

These cells may be cells into which a foreign gene encoding a protein desired to be expressed (for example, an antibody) has been introduced. The cell is preferably a cell that produces an antibody. An expression vector can be used for introducing a foreign gene encoding a protein desired to be expressed, into a cell. An expression vector containing a DNA encoding a protein desired to be expressed, an expression control sequence (for example, an enhancer, a promoter, a terminator, or the like), and a selection marker gene as desired is introduced into a cell, whereby it is possible to prepare a cell into which a foreign gene encoding a protein desired to be expressed is introduced. The expression vector is not particularly limited and can be appropriately selected and used depending on the kind, use application, and the like of the cell.

As the promoter, it is possible to use any promoter of which the function can be exhibited in mammalian cells.

Examples thereof include a promoter of the immediate early (IE) gene of the cytomegalovirus (CMV), an early promoter of SV40, a retrovirus promoter, a metallothionein promoter, a heat shock promoter, an SRα promoter, and a promoter and enhancer of the Moloney murine leukemia virus. In addition, an enhancer of the IE gene of human CMV may be used together with the promoter.

As the selection marker gene, it is possible to use, for example, a drug resistance gene (a neomycin resistance gene, a dihydrofolate reductase (DHFR) gene, a puromycin resistance gene, a blasticidin resistance gene, a hygromycin resistance gene, a cycloheximide resistance gene, or the like), or a fluorescence gene (a gene encoding a green fluorescent protein GFP or the like).

The method of introducing an expression vector into a cell is not particularly limited, and it is possible to use, for example, a calcium phosphate method, an electroporation method, a liposome method, a gene gun method, or a lipofection method.

The production method for a product according to the embodiment of the present invention includes culturing a cell using a cell culture device to produce a product from the cell.

According to the present invention, there is provided a product that is produced by the product production method for a product according to the embodiment of the present invention.

In the present invention, the kind of the product is not particularly limited; however, it is preferably a protein and more preferably a recombinant protein. Examples of the product include a recombinant polypeptide chain, a recombinant secreted polypeptide chain, an antigen-binding protein, an antibody (for example, a human antibody, a humanized antibody, a chimeric antibody, a mouse antibody, or a bispecific antibody), an Fc fusion protein, a fragmented immunoglobulin, and a single-chain antibody (scFv). In addition, it may be, in addition to the above, an adenovirus, an adeno-associated virus, a lentivirus, or the like.

The product is preferably an antibody, and more preferably a human antibody, a humanized antibody, a chimeric antibody, or a mouse antibody. Examples of the fragmented immunoglobulin include Fab, F(ab')2, and Fv. The class of the antibody is also not particularly limited, and it may be any class of IgG such as IgG1, IgG2, IgG3, or IgG4, IgA, IgD, IgE, or IgM. However, IgG or IgM is preferable in a case of being used as a medicine.

The human antibody includes all antibodies having one or a plurality of variable and constant regions induced from human immunoglobulin sequences. In one embodiment, all variable and constant domains are induced from human immunoglobulin sequences (complete human antibodies).

In a case of being administered to a human subject, the humanized antibody has a sequence different from a sequence of an antibody induced from a non-human species by substitution, deletion, and/or addition of one or a plurality of amino acids so that there is a low possibility for the humanized antibody to induce an immune response and/or so that induction of a severe immune response is reduced as compared with the antibody of non-human species. In one example, specific amino acids in a framework and constant domains of heavy chains and/or light chains of an antibody of non-human species are mutated to produce a humanized antibody. In another example, a constant domain from a human antibody is fused to a variable domain of an antibody of a non-human species.

The chimeric antibody is an antibody in which variable regions and constant regions having origins different from each other are linked. For example, an antibody consisting of variable regions of heavy chains and light chains of a mouse antibody and consisting of constant regions of heavy chains and light chains of a human antibody is a mouse/human heterologous chimeric antibody. It is possible to prepare a recombinant vector expressing a chimeric antibody by linking a DNA encoding variable regions of a mouse antibody and a DNA encoding constant regions of a human antibody and then incorporating the linked DNA into an expression vector. It is possible to acquire a chimeric antibody produced during the culturing by culturing a recombinant cell transformed with the above vector and expressing the incorporated DNA.

The bispecific antibody is an antibody that recognizes two kinds of antigenic specificity, which are different from each other. Various forms of bispecific antibodies are present. As a method of preparing a bispecific antibody, it has been reported a method of preparing a bispecific antibody by binding two immunoglobulin molecules by using a crosslinking agent such as N-succinimidyl 3-(2-pyridyldithiol)propionate or S-acetylmercaptosuccinic acid anhydride, a method of preparing a bispecific antibody by binding Fab fragments of immunoglobulin molecules to each other. In addition, a bispecific antibody can be expressed by introducing a gene encoding the bispecific antibody into a cell.

The Fc fusion protein indicates a protein having an Fc region and includes an antibody.

The Fab is a monovalent fragment having VL, VH, CL, and CH1 domains.

The F(ab')2 is a divalent fragment having two Fab fragments bound by a disulfide crosslinking at a hinge region.

The Fv fragment has VL and VH domains of a single arm of an antibody.

The single-chain antibody (scFv) is an antibody in which VL and VH regions are joined through a linker (for example, a synthetic sequence of amino acid residues) to form a continuous protein chain, where the linker is long enough to allow the protein chain to fold for itself and form a monovalent antigen binding site.

The antibody is not particularly limited; however, examples thereof include an anti-IL-6 receptor antibody, an anti-IL-6 antibody, an anti-glypican-3 antibody, an anti-CD3 antibody, an anti-CD20 antibody, an anti-GPIIb/IIIa antibody, an anti-TNF antibody, an anti-CD25 antibody, an anti-EGFR antibody, an anti-Her2/neu antibody, an anti-RSV antibody, an anti-CD33 antibody, an anti-CD52 antibody, an anti-IgE antibody, an anti-CD11a antibody, an anti-VEGF antibody, and an anti-VLA4 antibody.

Regarding the product recovery, the culture solution may be simply recovered. For example, a liquid obtained by removing at least a part of the cells from the culture solution using a filter or a centrifuge may be recovered, and a known method is used without particular limitation. In a case of desiring to improve the purity of the product, change the solvent of the product, or change the form of the product to, for example, a power form, the culture solution or the liquid can be subjected to further treatment.

In addition, a part of the culture solution can be recovered while being perfused, or a part of the culture solution can be recovered as a liquid which is obtained by removing at least a part of cells from the part of the culture solution, which is recovered by using a filter or a centrifuge while being perfused.

The product can be purified by a purification treatment. The obtained product can be purified to high purity. For the separation and purification of the product, the separation and purification methods that are used for general proteins may be used. For example, it is possible to carry out separation and purification of a product by appropriately selecting and combining means such as chromatography such as affinity chromatography, a filter, ultrafiltration, salting out, dialysis, sodium dodecyl sulfate (SDS) polyacrylamide gel electrophoresis, isoelectric focusing electrophoresis, and the like, which are not limited thereto. The concentration of the product obtained as above can be measured according to absorbance measurement, enzyme-linked immunosorbent assay (ELISA), or the like. In addition, in a case where the product is an antibody, the titer of the antibody can also be measured with a commercially available analytical instrument such as Cedex Bio manufactured by F. Hoffmann-La Roche, Ltd.

Examples of the column that is used for affinity chromatography include a protein A column and a protein G column. Examples of the chromatography other than affinity chromatography include ion exchange chromatography, hydrophobic chromatography, gel filtration, reverse phase chromatography, and adsorption chromatography. The chromatography can be carried out using liquid phase chromatography such as high performance liquid chromatography (HPLC) or fast protein liquid chromatography (FPLC).

It is noted that it is also possible to modify the product or partially remove a peptide thereof by allowing an appropriate polypeptide modifying enzyme to act on the useful substance before or after purification. As the polypeptide modifying enzyme, for example, trypsin, chymotrypsin, lysyl endopeptidase, protein kinase, or glucosidase is used.

The product that is produced according to the present invention can be used, for example, for a biopharmaceutical drug, or regenerative medicine.

The present invention will be more specifically described using the following examples; however, it is not limited by the examples.

Examples

<Establishment of antibody-producing cell>

A vector containing a nucleic acid sequence encoding each of IgG1 and IgG4 was constructed, and the constructed vector was introduced into a CHO-DG44 cell to prepare a CHO-DG44 cell expressing IgG1 (an IgG1 cell) and a CHO-DG44 cell expressing IgG4 (an IgG4 cell). The construction of the vector and the introduction thereof into the cell were carried out according to Example 2 of JP2016-517691A. As described above, CHO cells producing monoclonal antibodies were prepared and used in the following experiment.

<Cell culture>

In Examples and Comparative Examples, experiments were carried out using the cell culture device shown in Fig. 1. Regarding the outline thereof, the CHO cells that produce an antibody by perfusion culture were proliferated up to $120 \times 10^6$ cells/mL, cell bleeding was started at the time when the cell density reached $120 \times 10^6$ cells/mL, and the number of days taken for the clogging of the hollow fiber membrane and the like were evaluated. The details are as follows.

1 L of a culture medium was charged into a 2 L glass culture tank (product name, manufactured by ABLE Corporation). The culture medium was CD OptiCHO (manufactured by Thermo Fisher Scientific, Inc.), and 0.9% by mass of a poloxamer (Poloxamer-188) was added to this culture medium.

On the 0th day, cells were seeded at $0.5 \times 10^6$ cells/mL.

At a stirring rotation speed of 150 rpm, 38 mL/min of air and 2 mL/min of $CO_2$ were supplied from the upper surface, and from the bottom surface, $O_2$ was supplied by automatic control from a sparger installed in the culture tank so that the oxygen concentration in the culture solution was 80%.

The pH in the culture tank was read so that the pH in the culture tank was 7.0, and then an aqueous solution of 1.0 mol/L $NaHCO_3$ was automatically added thereto in a case where the pH was less than 7.0, whereby the pH control was started.

After culturing for 2 days after seeding, the cell culture solution was continuously filtered with ATF2 manufactured by Repligen Corporation, and the recovery liquid was recovered while continuously supplying a culture medium at a perfusion ratio of 0.8 vvd.

Further, on the 5th day, the perfusion ratio was changed to 1.6 vvd.

Further, on the 6th day, the addition rate of dimethicone in the anti-foaming agent was changed to 7.2 mg/day/L.

Further, on the 7th day, the pH of the culture solution decreases, and the aqueous alkali solution automatically starts to be added to the culture solution.

Further, on the 9th day to the 11th day, in a case where the cell density reached $120 \times 10^6$ cells/mL, the cell bleeding was carried out while a part of the culture solution was drained so that the cell density was maintained at $120 \times 10^6$ cells/mL.

The culture was continued until the membrane clogging occurred and the ATF could not operate.

The culture medium to be used, the addition rate of dimethicone, the pH control, and the like were changed as shown in the table below to carry out a plurality of experiments.

<Evaluation method>

(1) Measurement of cell density and viability

The culture solution in the culture tank was extracted and subjected to measurement using Cell Viability Analyzer Vi-cell XR manufactured by Beckman Coulter, Inc. It is noted that for the Vi-cell software, Vi-cell XR2.04 was used, and the parameters at the time of measurement were set as follows.

Min diameter: 6 $\mu$m,
Max diameter: 50 $\mu$m

Dilution: In a case where the cell concentration was $10 \times 10^6$ cells/mL or less, the sample was not diluted, and the Dilution was set to 1.

In a case where the cell concentration was higher than $10 \times 10^6$ cells/mL, the sample was diluted 10 times, and the Dilution was set to 10.

Cell brightness: 75%,
Cell sharpness: 100
Viable cell spot brightness: 75%
Viable cell spot area: 5%
Minimum circularity: 0
Decluster degree: Medium

(2) Measurement of pH and dissolved $CO_2$

The culture solution in the culture tank was extracted and subjected to measurement using RAPIDLab 348EX, manufactured by SIEMENS AG. The carbon dioxide ($CO_2$) flow rate of the air supply pipe was adjusted so that the dissolved $CO_2$ was 120 mmHg in the production period.

(3) Measurement of fine particle (debris) density

The culture solution in the culture tank was extracted and diluted 10,000 times with ISOTON (Beckman Coulter, Inc.). The diluted culture solution was subjected to a measurement of a particle size distribution in 1.46 to 20 $\mu$m with a precise particle size distribution measuring device Multisizer 4e (Beckman Coulter, Inc.).

(4) Measurement of lactate dehydrogenase (LDH)

The culture solution was extracted, and the cells and the supernatant were separated from each other at 300 G/5 minutes. The supernatant was subjected to measurement with Cedex Bio manufactured by F. Hoffmann-La Roche, Ltd.

(5) Measurement of antibody quality

The culture solution in the culture tank was extracted, and the cells were filtered with Whatman (pore size: 0.2 $\mu$m, diameter: 25 mm) manufactured by Cytiva. The above-described culture supernatant was loaded onto a MabSelect Sure column equilibrated at a pH of 6.0, and the antibody was eluted at a pH of 3.2.

For the evaluation of antibody charge, the antibody recovery liquid described above was subjected to measurement by cation exchange high performance liquid chromatography (HPLC).

For the evaluation of mannose 5 (M5), the antibody in the antibody recovery liquid obtained was subjected to a digestion treatment with peptide-N-glycosidase F to cut out the N-type sugar chain, and fluorescent labeling was carried out with 2-aminobenzamide.

M5 of the treated antibody was measured by a C18 (ODS) reverse phase HPLC column.

(6) Evaluation standards

The evaluation standards for each evaluation are shown below.

Evaluation of membrane clogging:

S: A case where the number of days taken from the start of culture to the clogging is 30 days or more, the number of days taken from the start of perfusion to the clogging is 28 days or more, and the number of days taken from the start of cell bleeding to the clogging is 20 days or more.

A: A case where the number of days taken from the start of culture to the clogging is 20 to 29 days, the number of days taken from the start of perfusion to the clogging is 18 to 27 days, and the number of days taken from the start of cell bleeding to the clogging is 10 to 19 days.

B: A case where the number of days taken from the start of culture to the clogging is 15 to 19 days, the number of days taken from the start of perfusion to the clogging is 13 to 17 days, and the number of days taken from the start of cell bleeding to the clogging is 5 to 9 days.

C: A case where the number of days taken from the start of culture to the clogging is 14 days or less, the number of days taken from the start of perfusion to the clogging is 12 days or less, and the number of days taken from the start of cell bleeding to the clogging is 4 days or less.

Evaluation of antibody charge:

A: A case where both of the following facts are satisfied: the proportion $X$ of the area of the acidic peak satisfies $14\% \leq X \leq 22\%$, and the proportion $X$ of the area of the basic peak satisfies $11\% \leq X \leq 19\%$.

B: A case that corresponds to any one or more of the following facts: the proportion $X$ of the area of the acidic peak satisfies $10\% \leq X < 14\%$ or $22\% < X \leq 26\%$, and the proportion $X$ of the area of the basic peak satisfies $6\% \leq X < 11\%$ or $19\% < X \leq 23\%$.

C: A case that corresponds to any one or more of the following facts: the proportion $X$ of the area of the acidic peak satisfies $X < 10\%$ or $26\% < X$, and the proportion $X$ of the area of the basic peak satisfies $X < 6\%$ or $23\% < X$.

The proportion of the peak area is a proportion of the peak area of the acidic component in the chromatography or the peak area of the basic component in the chromatography with respect to the total peak area of the entire chromatography.

Evaluation of M5 of antibody:

A: The proportion $X$ of the peak area of M5 satisfies $0\% \leq X \leq 5\%$.
B: The proportion $X$ of the peak area of M5 satisfies $5\% < X \leq 6\%$.
C: The proportion $X$ of the peak area of M5 satisfies $6\% < X$.

The proportion of the peak area is a proportion of the peak area of the M5 (Mannose 5) component in the chromatography with respect to the total peak area of the entire chromatography.

<Results of measurement and evaluation>

The results of the measurement and the evaluation are shown in Table 2 and Table 3.

As shown in Table 3, it has been confirmed that the quality of the produced antibody is good in Examples 5, 10, and 11.

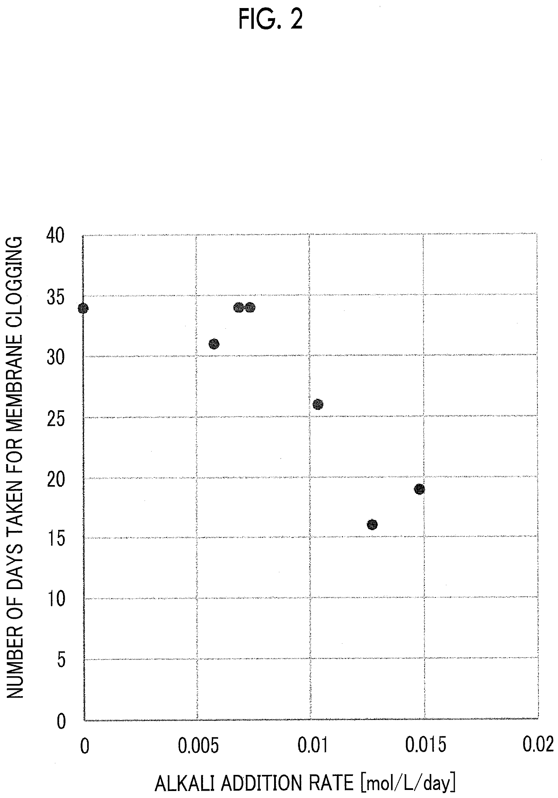

Fig. 2 shows the relationship between the alkali addition rate and the number of days taken until membrane clogging occurs, in Examples 1 to 3, 5, and 7 to 9. From the results in Fig. 2, it can be seen that the membrane clogging can be suppressed by reducing the alkali addition rate.

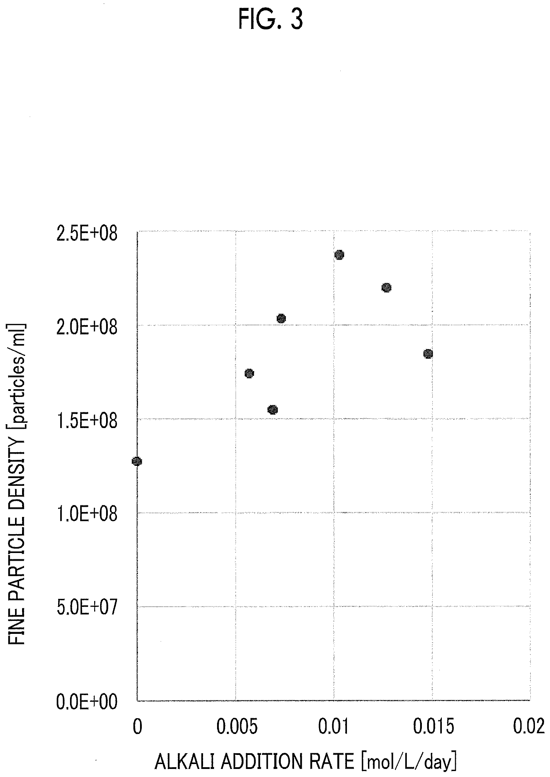

Fig. 3 shows the relationship between the alkali addition rate and the fine particle density, in Examples 1 to 3, 5, and 7 to 9. The fine particle density was defined as the cumulative number of densities of cells of 1 to 6 $\mu$m. The fine particles are debris/fragments derived from cells (14 $\mu$m) and serve as an indicator of cell damage. From the results in Fig. 3, it can be seen that the cell damage can be suppressed by reducing the alkali addition rate.

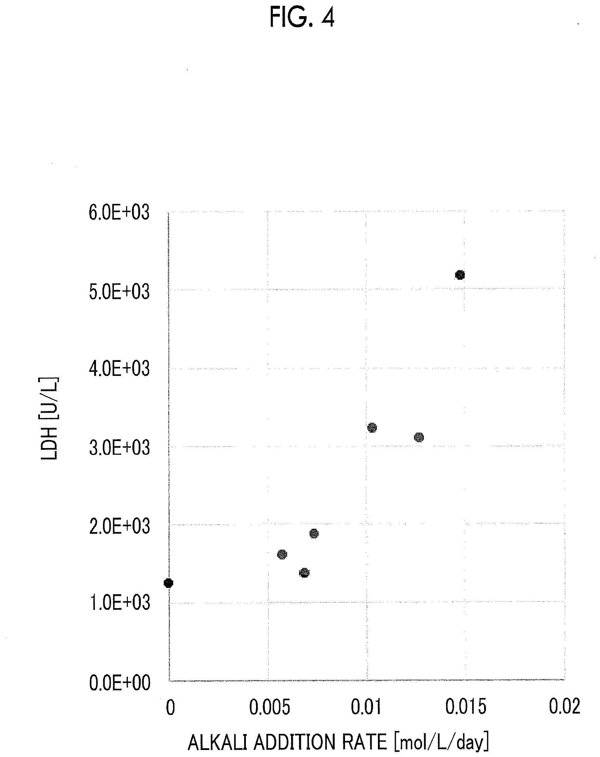

Fig. 4 shows the relationship between the alkali addition rate and the LDH, in Examples 1 to 3, 5, and 7 to 9. LDH is an enzyme component that leaks from damaged cells and is generally used as an indicator of cell damage. From the results in Fig. 4, it can be seen that the cell damage can be suppressed by reducing the alkali addition rate.

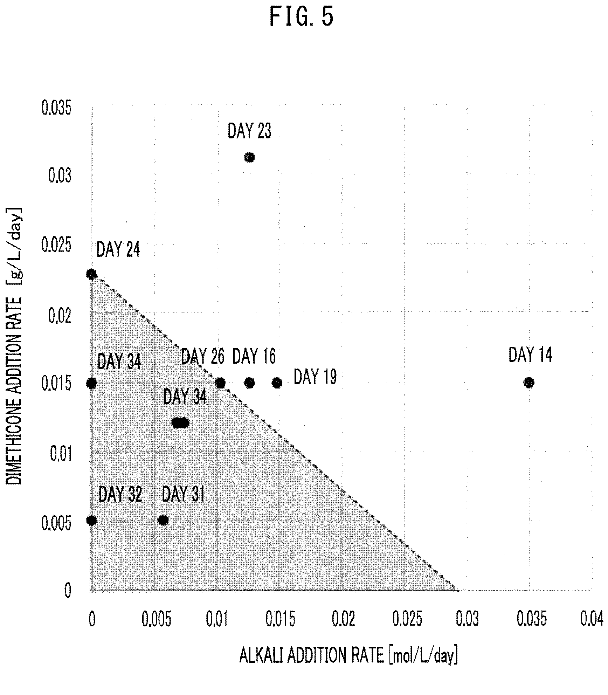

In addition, Fig. 5 shows the relationship between the alkali addition rate, the anti-foaming agent addition rate, and the number of days taken until membrane clogging occurs, in Examples 1 to 9 and 11, and Comparative Example 1. It can be seen that the higher the anti-foaming agent addition rate is and the higher the alkali addition rate is, the more easily the membrane clogging occurs. A region, in which X [mol/L/day], which is an addition rate of the alkali, and B [g/L/day], which is an addition rate of dimethicone which is an anti-foaming agent, satisfy B < -0.79X + 0.0228, is a region that has the evaluation of membrane clogging of S.

[Table 1]

| | Cell density $\times 10^6$ cells/ml | Culture medium | | | | Alkali | | | Anti-foaming agent (dimethicone) | Total amount of NaHCO3 (mol/L/day) |
|---|---|---|---|---|---|---|---|---|---|---|
| | | pH | $NaHCO_3$ concentration (mol/L) | Adding amount of culture medium per day (vvd) | NaHCO3 (mol/L/day) | pH | $NaHCO_3$ concentration (mol/L) | NaHCO3 (mol/L/day) | Addition rate g/L/day | |
| Example 1 | 120 | 7.4 | 0.02380669 | 1.2 | 0.028568028 | 8.5 | 1 | 0 | 0.01488 | 0.028568028 |
| Example 2 | 120 | 7.4 | 0.02380669 | 1.2 | 0.028568028 | 8.5 | 1 | 0.012704727 | 0.01488 | 0.041272755 |
| Example 3 | 120 | 7.4 | 0.02380669 | 1.2 | 0.028568028 | 8.5 | 1 | 0.010335359 | 0.01488 | 0.038903387 |
| Example 4 | 120 | 7.4 | 0.02380669 | 1.2 | 0.028568028 | 8.5 | 1 | 0 | 0.00504 | 0.028568028 |
| Example 5 | 120 | 7.4 | 0.02380669 | 1.2 | 0.028568028 | 8.5 | 1 | 0.006892854 | 0.012 | 0.035460882 |
| Example 6 | 120 | 7.4 | 0.02380669 | 1.2 | 0.028568028 | 8.5 | 1 | 0.012650178 | 0.0312 | 0.041218206 |
| Example 7 | 120 | 7.2 | 0.010201167 | 1.2 | 0.0122414 | 8.5 | 1 | 0.014804876 | 0.01488 | 0.027046276 |
| Example 8 | 120 | 7.4 | 0.02380669 | 1.2 | 0.028568028 | 8.5 | 1 | 0.007366625 | 0.012 | 0.035934652 |
| Example 9 | 120 | 7.4 | 0.02380669 | 1.2 | 0.028568028 | 8.5 | 1 | 0.00576506 | 0.00504 | 0.034333088 |
| Comparative Example 1 | 120 | 7.4 | 0.02380669 | 1.2 | 0.028568028 | 8.5 | 1 | 0.035 | 0.01488 | 0.063568028 |

[Table 2]

| | Membrane clogging | | | | Average pH | Minimum pH | Fine particle density (1 to 6 μm in total ) (particles/mL) | Viability (%) | LDH (U/L) |
|---|---|---|---|---|---|---|---|---|---|
| | Number of days taken from start of culture to clogging | Number of days taken from start of perfusion to clogging | Number of days taken from production period to clogging | Evaluation | | | | | |
| Example 1 | 34 | 32 | 24 | S | 6.89 | 6.83 | 1.3E+08 | 95.9 | 1251.4 |
| Example 2 | 16 | 14 | 6 | B | 6.96 | 6.92 | 2.1E+08 | 95.4 | 3100.614 |
| Example 3 | 26 | 24 | 16 | A | 6.94 | 6.91 | 2.4E+08 | 95.3 | 3222.917 |
| Example 4 | 32 | 30 | 22 | A | 6.88 | 6.82 | - | - | - |
| Example 5 | 34 | 32 | 24 | S | 6.92 | 6.90 | 1.5E+08 | 96.2 | 1374.183 |
| Example 6 | 23 | 21 | 13 | A | 6.93 | 6.90 | - | - | - |
| Example 7 | 19 | 17 | 9 | B | 6.86 | 6.84 | 1.8E+08 | 92.4 | 5172.14 |
| Example 8 | 34 | 32 | 24 | S | 6.91 | 6.89 | 2.0E+08 | 95.4 | 1867.038 |
| Example 9 | 31 | 29 | 21 | A | 6.90 | 6.78 | 1.7E+08 | 95.7 | 1608.909 |
| Comparative Example 1 | 14 | 12 | 4 | C | 7.02 | 6.95 | - | - | - |

EP 4 502 149 A1

[Table 3]

| | Cell density $\times 10^6$ cells/ml | pH | NaHCO$_3$ concentration (mol/L) | Adding amount of culture medium per day (L/day/L-WV) | NaHCO3 mol/day | Average pH | Minimum pH | Evaluation of charge | Evaluation of sugar chain | Charge | | Sugar chain |
|---|---|---|---|---|---|---|---|---|---|---|---|---|
| | | | | | | | | | | Acidic | Basic | M5 |
| Example 10 | 120 | 7.2 | 0.010201167 | 1.2 | 0.0122414 | 6.75 | 6.70 | B | B | 11% | 21% | 5.5% |
| Example 11 | 120 | 7.3 | 0.01700988 | 1.2 | 0.020411856 | 6.83 | 6.77 | A | A | 15% | 18% | 2.5% |
| Example 5 | 120 | 7.4 | 0.02380669 | 1.2 | 0.028568028 | 6.92 | 6.90 | A | A | 14% | 17% | 1.5% |

Explanation of References

1: culture medium supply pipe
2: anti-foaming agent supply pipe
3: alkali supply pipe
4: air supply pipe
5: sparger air supply pipe6: exhaust pipe
7: sampling tube
8: pH sensor
9: dissolved oxygen sensor
10: pressure sensor
11: pressure sensor
12: hollow fiber membrane
13: pressure sensor
14: culture container
15: sparger
16: stirring blade

## Claims

1. A production method for a product, comprising:

   culturing cells,
   wherein the production method for a product is perfusion culture in which a cell density at a time of production of the product is $80 \times 10^6$ cells/mL or more and $300 \times 10^6$ cells/mL or less, where a period of the perfusion culture is 13 days or more and 500 days or less, and
   in a period of at least one day or more in the period of the perfusion culture, X [mol/L/day], which is an alkali addition rate per day, is controlled in a range of $0 \leq X < 0.029$, where a pH of an aqueous alkali solution to be added satisfies $7 < pH < 13$.

2. The production method for a product according to claim 1,
   wherein the cell density in the perfusion culture is $100 \times 10^6$ cells/mL or more and $300 \times 10^6$ cells/mL or less.

3. The production method for a product according to claim 1 or 2,
   wherein the period of the perfusion culture is 18 days or more and 500 days or less.

4. The production method for a product according to any one of claims 1 to 3,
   wherein a period of culture for the production of the product is 5 days or more and 450 days or less.

5. The production method for a product according to any one of claims 1 to 4,
   wherein in a period of at least 13 days or more in the period of the perfusion culture, X [mol/L/day], which is the alkali addition rate per day, is controlled in a range of $0 \leq X < 0.008$.

6. The production method for a product according to any one of claims 1 to 5,
   wherein in a period of at least 13 days or more in the period of the perfusion culture, X [mol/L/day], which is the alkali addition rate per day, is 0.

7. The production method for a product according to any one of claims 1 to 6,
   wherein in a period of 50% or more of the perfusion culture, X [mol/L/day], which is the alkali addition rate per day, is controlled in a range of $0 \leq X < 0.029$.

8. The production method for a product according to any one of claims 1 to 7,
   wherein a continuous separation method for a cell culture solution in the perfusion culture is membrane filtration.

9. The production method for a product according to claim 8,

wherein the membrane filtration is alternating tangential flow filtration, which is referred to as ATF.

10. The production method for a product according to claim 8 or 9,
wherein Y [L/m$^2$/hour], which is a flux during filtration, satisfies $0 < Y \leq 10$.

11. The production method for a product according to any one of claims 1 to 10,
wherein an average pH of a culture solution during the culturing is 6.7 to 7.2.

12. The production method for a product according to any one of claims 1 to 11,
wherein a minimum pH of a culture solution during the culturing is 6.6 or more.

13. The production method for a product according to any one of claims 1 to 12,
wherein a pH of a culture solution during the culturing is controlled by automatically adding an aqueous alkali solution while subjecting a pH in the culture solution to an in-line measurement.

14. The production method for a product according to any one of claims 1 to 13,
wherein in the perfusion culture, after a cell density reaches a target cell density, which is $80 \times 10^6$ cells/mL or more, a culture solution containing cells is extracted to maintain the cell density within $\pm$ 40% of the target cell density, and the product is recovered in a period in which the cell density is maintained within $\pm$ 40% of the target cell density.

15. The production method for a product according to any one of claims 1 to 13, further comprising:
extracting a culture solution containing cells at least once or more a day in the perfusion culture after a cell density reaches a target cell density, which is $80 \times 10^6$ cells/mL or more, and then adjusting the cell density to be within $\pm$ 10% of the target cell density.

16. The production method for a product according to any one of claims 1 to 15,
wherein a dissolved $CO_2$ concentration in a period in which the cell density is maintained at $80 \times 10^6$ cells/mL or more is 60 to 180 mmHg.

17. The production method for a product according to any one of claims 1 to 16,
wherein the alkali is $NaHCO_3$ and/or $Na_2CO_3$.

18. The production method for a product according to any one of claims 1 to 17,
wherein the alkali is $NaHCO_3$.

19. The production method for a product according to any one of claims 1 to 18,
wherein a concentration Z [mol/L] of the aqueous alkali solution satisfies $0 < Z < 5$.

20. The production method for a product according to any one of claims 1 to 19,
wherein a pH of a culture medium to be added in the perfusion culture is 7.0 to 8.0.

21. The production method for a product according to any one of claims 1 to 20,
wherein in a case where a pH of a culture solution decreases in a case where the culturing is carried out without adding an alkali, a culture medium to which an alkali is added is supplied to control the pH of the culture solution.

22. The production method for a product according to any one of claims 1 to 21, further comprising:
supplying a culture medium to which an alkali of $1.0 \times 10^{-3}$ to 2.0 mol/L is added, in a case where a pH of a culture solution is less than 6.85.

23. The production method for a product according to any one of claims 1 to 22,

wherein an anti-foaming agent containing dimethicone is added in the perfusion culture, and
X [mol/L/day], which is an addition rate of the alkali, and B [g/L/day], which is an addition rate of the dimethicone, satisfy $B < -0.79X + 0.0228$.

24. The production method for a product according to any one of claims 1 to 23,
wherein alkali addition is not carried out in an entire period of the perfusion culture.

**25.** The production method for a product according to any one of claims 1 to 24, wherein the cell is an animal cell.

**26.** The production method for a product according to any one of claims 1 to 25, wherein the cell is a CHO cell.

**27.** The production method for a product according to any one of claims 1 to 26, wherein the product is an antibody.

**28.** A product that is produced by the production method for a product according to any one of claims 1 to 27.

# FIG. 1

# FIG. 2

# FIG. 3

# FIG. 4

FIG. 5

<table>
<tr><td colspan="2" style="text-align:center">**INTERNATIONAL SEARCH REPORT**</td><td colspan="2">International application No.<br>**PCT/JP2023/013100**</td></tr>
</table>

**A.    CLASSIFICATION OF SUBJECT MATTER**

*C12N 5/10*(2006.01)i; *C12P 21/08*(2006.01)i
FI:    C12P21/08; C12N5/10

According to International Patent Classification (IPC) or to both national classification and IPC

**B.    FIELDS SEARCHED**

Minimum documentation searched (classification system followed by classification symbols)

C12N5/10; C12P21/08

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C.    DOCUMENTS CONSIDERED TO BE RELEVANT**

| Category* | Citation of document, with indication, where appropriate, of the relevant passages | Relevant to claim No. |
|---|---|---|
| X | WO 2019/181234 A1 (FUJIFILM CORP.) 26 September 2019 (2019-09-26) claims 1, 3-5, paragraphs [0024], [0028], [0037], [0075], examples | 1-9, 11-15, 17-22, 24-28 |
| Y | claims 1, 3-5, paragraphs [0024], [0028], [0037], [0075], examples | 1-28 |
| Y | JP 2019-523002 A (REPLIGEN CORP.) 22 August 2019 (2019-08-22) claims 1, 13, paragraph [0009] | 1-28 |
| Y | JP 2021-536262 A (MOMENTA PHARMACEUTICALS INC.) 27 December 2021 (2021-12-27) paragraph [0017] | 1-28 |
| Y | WO 2021/143699 A1 (WUXI BIOLOGICS (SHANGHAI) CO., LTD.) 22 July 2021 (2021-07-22) paragraph [0096] | 1-28 |
| Y | JP 3-117494 A (UNILEVER NAAMLOZE VENNOOTSCHAP) 20 May 1991 (1991-05-20) p. 6, lower right column, lines 6-10 | 1-28 |
| Y | JP 2021-518148 A (SANOFI PASTEUR, INC.) 02 August 2021 (2021-08-02) paragraph [0036] | 1-28 |

☐ Further documents are listed in the continuation of Box C.        ☑ See patent family annex.

| *    Special categories of cited documents: | |
|---|---|
| "A"    document defining the general state of the art which is not considered to be of particular relevance | "T"    later document published after the international filing date or priority date and not in conflict with the application but cited to understand the principle or theory underlying the invention |
| "E"    earlier application or patent but published on or after the international filing date | "X"    document of particular relevance; the claimed invention cannot be considered novel or cannot be considered to involve an inventive step when the document is taken alone |
| "L"    document which may throw doubts on priority claim(s) or which is cited to establish the publication date of another citation or other special reason (as specified) | "Y"    document of particular relevance; the claimed invention cannot be considered to involve an inventive step when the document is combined with one or more other such documents, such combination being obvious to a person skilled in the art |
| "O"    document referring to an oral disclosure, use, exhibition or other means | |
| "P"    document published prior to the international filing date but later than the priority date claimed | "&"    document member of the same patent family |

| Date of the actual completion of the international search | Date of mailing of the international search report |
|---|---|
| **08 June 2023** | **20 June 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

INTERNATIONAL SEARCH REPORT
Information on patent family members

International application No.

**PCT/JP2023/013100**

| Patent document cited in search report | | | Publication date (day/month/year) | Patent family member(s) | | | Publication date (day/month/year) |
|---|---|---|---|---|---|---|---|
| WO | 2019/181234 | A1 | 26 September 2019 | US 2020/0399585 A1 claims 1, 3-5, paragraphs [0057], [0063], [0080], [0146], examples | | | |
| JP | 2019-523002 | A | 22 August 2019 | US 2018/0148677 A1 claims 1, 13, paragraph [0010] | | | |
| JP | 2021-536262 | A | 27 December 2021 | US 2021/0222109 A1 paragraph [0017] | | | |
| WO | 2021/143699 | A1 | 22 July 2021 | EP 4090731 A1 | | | |
| JP | 3-117494 | A | 20 May 1991 | US 5789212 A column 5, lines 29-32 | | | |
| JP | 2021-518148 | A | 02 August 2021 | US 2020/0407678 A1 paragraph [0038] | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016534732 A
- JP 2021503916 A
- JP 2014500032 A
- JP 2016517691 A